# DEMANDE DE BREVET EUROPEEN

(11) **EP 0 545 821 A1**
(43) Date de publication de la demande: **09.06.1993**
(21) Numéro de dépôt: 92403268.3
(22) Date de dépôt: 03.12.1992
(51) Int. Cl.: A61G 3/00

(54) **Unité de clinique vétérinaire mobile**

(30) Priorité: 03.12.1991 ES 9103659
(71) Demandeur: Ruperez Crespo, Roberto, E-31500 Tudela (Navarra) (ES)
(72) Inventeur: Sancho Claver, Antonio, E-22760 Bailo (Huesca) (ES)
(74) Mandataire: Hasenrader, Hubert

(57) **Abrégé**

Ce module (2) est essentiellement composé d'un caisson pouvant se placer sur la plate-forme d'un véhicule (1) (camionnette ou équivalent) et qui comporte : une portière arrière (3) donnant accès à des bacs ou tiroirs (5), et des portières supérieures (4) donnant accès à des bacs compartimentés, ces tiroirs et bacs pouvant contenir des médicaments, produits, outils, équipements, etc.. Le module (2) comporte aussi des réservoirs d'eau chaude et d'eau froide équipés d'électropompes et de tuyaux de projection, la portière arrière (3) pouvant servir de table d'opération.

## Description

Les activités vétérinaires exigent très fréquemment le déplacement des vétérinaires pour soigner les animaux sur le lieu même où ils se trouvent, y compris dans les champs, loin de toute installation adéquate, de sorte que les vétérinaires ont l'habitude de se déplacer avec une simple trousse qui contient les instruments et produits les plus élémentaires, avec lesquels ils peuvent uniquement réaliser de simples examens superficiels et de purs traitements de premier secours, ceci étant évidemment insuffisant et précaire dans de nombreux cas puisque, s'il existe des circonstances de plus grande gravité, le problème ne peut pas être résolu convenablement, de sorte que, dans de nombreux cas, l'absence d'intervention à temps peut être la cause de conséquences irréversibles.

Pour résoudre efficacement ce problème et faire en même temps en sorte que l'activité en déplacement des vétérinaires soit plus pratique, plus commode et plus efficace, l'invention a pour objet une unité de clinique vétérinaire mobile qui, étant adaptable à un véhicule classique, peut être transportée facilement jusqu'à n'importe quel lieu accessible pour le véhicule porteur et qui comprend, dans un ensemble très simple et pratique, tout ce qui est nécessaire pour effectuer en ce lieu la consultation ou l'intervention voulue.

Cette unité objet de l'invention consiste essentiellement en un module en forme de caisson ou d'armoire, possédant la configuration et les formes necessaires pour s'adapter de façon appropriée à la zone de charge du véhicule auquel il est destiné à s'accoupler, ce module comprenant intérieurement des compartiments, bacs et espaces pour loger dans de bonnes conditions les instruments, appareils et produits nécessaires pour toute intervention vétérinaire, la partie arrière du module formant une portière qui recouvre l'accès à une série d'éléments qu'on peut en extraire par coulissement, et qui, en même temps, sert de table de travail en position ouverte, tandis que, dans la partie supérieure du module, sont formées des portes latérales pouvant basculer vers le haut, au droit d'accès respectifs à l'intérieur du module, où sont incorpores ou peuvent trouver place différents outils et appareils.

Ce module possède une alimentation en eau chaude et froide, fournie par des réservoirs formés dans sa propre unité, ainsi qu'un chauffage à fonctionnement indépendant ou pouvant en option se raccorder à celui du véhicule porteur, et il possède de même une chambre frigorifique raccordable au circuit électrique du véhicule ou à une prise électrique du réseau d'alimentation classique ; cependant que, pour les opérations de chargement sur le véhicule porteur correspondant, et de déchargement de ce véhicule, le module possède des points d'accrochage appropries qui permettent de déplacer ce module à l'aide d'un palan ou similaire.

On obtient de cette façon une unité simple, dans laquelle peut trouver place l'équipement le plus complet en instruments, appareils et produits qui permettra de procéder à des interventions vétérinaires de tout type dans des lieux écartés, ladite unité pouvant être commodément transportée dans un véhicule porteur sur lequel elle peut être installée d'une façon extrêmement facile, pour pouvoir être rendue entièrement fonctionnelle par accouplement audit véhicule.

L'unité objet de l'invention possède à coup sûr des caractéristiques très avantageuses qui lui confèrent une identité propre et un caractère préférable dans son utilisation pour la fonction à laquelle elle est destinée.

D'autres caractéristiques et avantages de l'invention seront mieux compris à la lecture de la description qui va suivre, d'un exemple de réalisation et en se référant aux dessins annexés, sur lesquels :

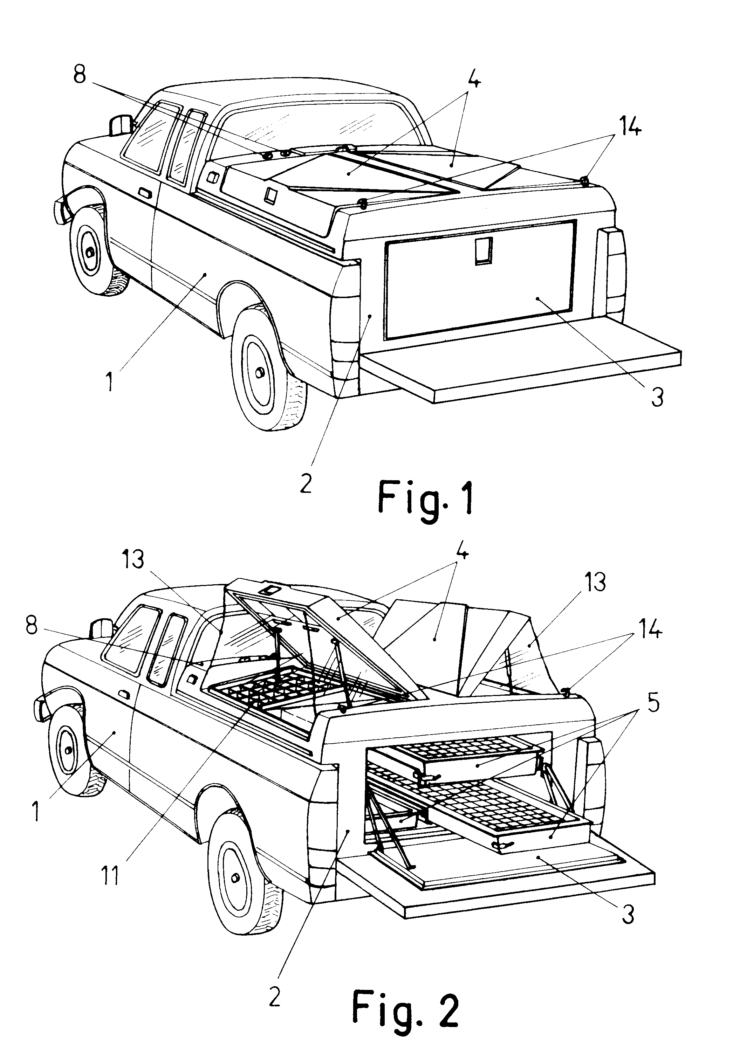

La figure 1 est une représentation en perspective de l'unité selon l'invention, montée sur un véhicule porteur correspondant, avec le module de cette unité totalement fermé.
La figure 2 est une représentation ressemblant à la précédente, mais avec les portières du module de l'unité ouvertes.

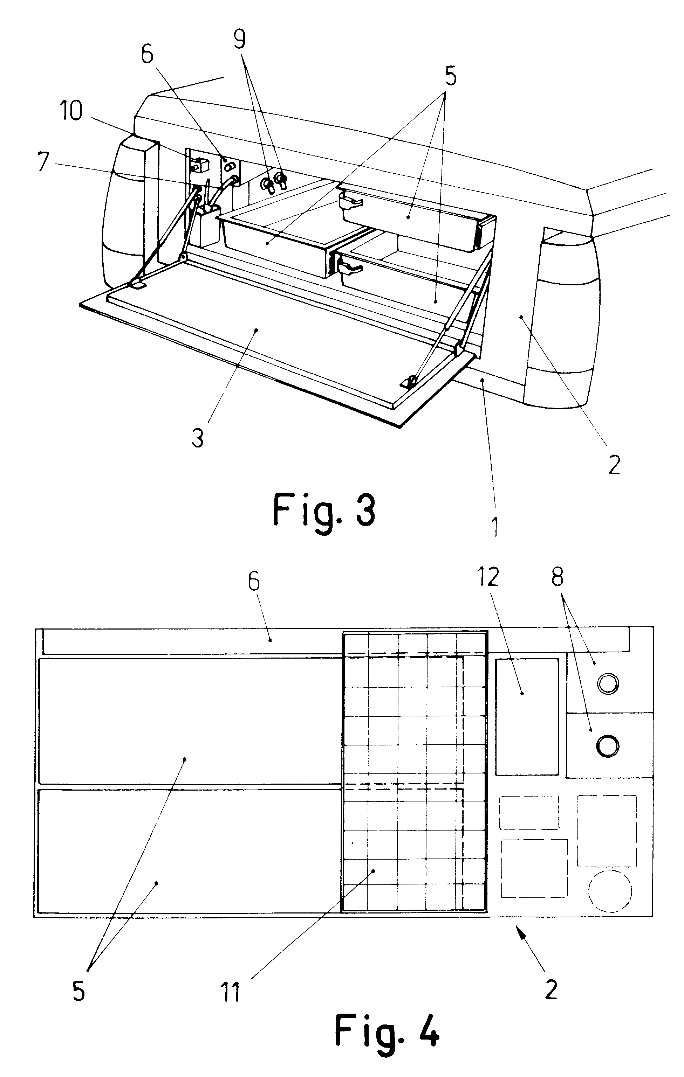

La figure 3 est une vue en perspective agrandie de la partie arrière du module, avec la portière ouverte, où l'on peut remarquer les éléments logés à l'intérieur.
La figure 4 est une représentation schématique de la distribution en plan de l'ensemble de l'unité en question.
La figure 5 est une représentation de l'unité sur un véhicule porteur, selon une réalisation de cette unité dans laquelle les portières supérieures sont disposées avec une pente sensible en partant d'une partie sommitale centrale plane, et équipées de mains-courantes périphériques sur leur surface ;
la figure 6 est une représentation en vue frontale arrière de cette réalisation précédente de l'unité, sur laquelle on peut voir les nervures d'appui dont elle est munie dans sa partie inférieure ;
la figure 7 est une représentation ressemblant à celle de la figure 5 mais montée sur un véhicule possédant un hayon de fermeture à l'arrière.

L'objet de l'invention est une unité de clinique vétérinaire mobile qui est prévue pour pouvoir être transportée en n'importe quel lieu, afin de permettre d'effectuer sur place tout acte ou intervention vétérinaire nécessaire, cette unité possédant tous les éléments nécessaires pour cela.

Cette unité peut être montée sur un véhicule porteur 1 par rapport auquel elle peut être constituée sous la forme d'un élément rapporté qui peut y être convenablement accouplé ou sous la forme d'une armoire pouvant être logée à l'intérieur ou encore sous la forme d'une partie carrossée du véhicule 1 lui-même.

Selon une forme de réalisation pratique préférée, mais non limitative, ladite unité consiste en un module 2 en forme de caisson, fabriqué en matière synthétique , qui est réalisé avec une configuration et les formes necessaires pour s'adapter convenablement à la zone de charge du véhicule 1 auquel il est destiné à être accouplé, possédant dans la partie arrière une portière rabattable 3, tandis que, dans sa partie supérieure, il comprend des portes latérales 4 qu'on peut basculer vers le haut pour les ouvrir.

La portière arrière 3 peut être rabattue jusqu'à prendre une position horizontale en position d'ouverture, de sorte que, dans cette position ouverte, ladite portière 3 sert de table de travail, en offrant la possibilité de l'utiliser comme table ou brancard d'opération , y compris pour interventions chirurgicales. La portière 3 donne accès à une baie d'où on peut extraire, à l'aide de robustes glissières, des éléments qui trouvent place à l'intérieur, de même que peuvent s'y loger une série de bacs 5 avantageusement prévus pour contenir tout type d'instruments et produits. Il est prévu que les bacs 5 comportent des séparations, formées par assemblage voulu de bandes disposées perpendiculairement entre elles, qui définissent des logements en forme de grille, dont l'utilisateur peut modifier à volonté le nombre, la dimension et la disposition en disposant sélectivement les bandes qui forment les grilles.

En ouvrant la portière 3, on donne accès à un logement 6 qui s'étend sur toute la longueur du module et dans lequel peuvent être logés des instruments de grande longueur de l'instrumentation vétérinaire tels que, par exemple, une sonde nasopharyngienne, un foetotome, un extracteur de foetus etc., ainsi qu'un ou plusieurs tuyaux 7 de projection d'eau, qui sont raccordés par l'intermédiaire d'une pompe électrique à des réservoirs correspondants 8 d'eau chaude et d'eau froide prévus dans la structure même du module 2, avec des robinets 9 pour ouvrir sélectivement la sortie d'eau à travers ledit tuyau 7. Il est prévu que les tuyaux 7 et les éléments de grande longueur qu'on fait sortir de cette façon sont reliés à un mécanisme de rentrée qui assure leur escamotage automatique dans le logement 6 lorsqu'on le désire.

La pompe électrique servant à projeter l'eau à partir des réservoirs 8 se branche pour son fonctionnement au circuit électrique du véhicule porteur 1 et, pour éviter qu'il puisse se produire accidentellement pendant le transport une mise en marche de la pompe, avec inondation consécutive de l'intérieur du module 2, on a prévu, dans l'encadrement de la portière 3, un interrupteur de sécurité 10 qui, lorsque la portière est fermée, est actionné par cette portière 3, en maintenant la pompe électrique débranchée, de sorte que, dans cette position fermée, il ne peut pas se produire de projection d'eau par le tuyau 7.

Les portières supérieures 4 correspondent à leur tour à des baies par lesquelles on peut accéder à l'intérieur du module 2 où, selon une forme de réalisation particulière et non limitative, il est prévu d'incorporer un bac transversal 11 pour loger également des instruments et produits, tandis que, à son tour, à l'intérieur du module, est formée une cavité dans laquelle peuvent être installés en position fixe certains appareils, de plus grand volume, tels qu'une chambre frigorifique 12 destinée à la conservation de produits périssables, en laissant ainsi libre un espace vide suffisant pour loger éventuellement provisoirement des appareils comme, éventuellement, une unité de radiographie, un appareil d'anesthésie, un vase à insémination ou tout autre élément de grande dimension.

Selon une caractéristique particulière, les portières supérieures 4 précitées sont munies, entre leur contour et la portée de fermeture, d'un store qui se place automatiquement en position et peut se replier sur lui-même lorsqu'on ouvre ou qu'on ferme lesdites portières 4, par exemple, une feuille de rideau 13 imperméable et transparente, munie d'accès qui ne laissent passer que les mains, de sorte qu'on évite l'entrée éventuelle d'eau de pluie et de souillures ambiantes à l'intérieur lorsque les portières 4 sont ouvertes.

L'unité peut être équipée d'une installation de chauffage, capable de fonctionner de façon independante grâce à des moyens électriques propres, ou bien par raccordement, par des tuyauteries, au chauffage du véhicule porteur 1, solutions qui peuvent co-exister, pour pouvoir être utilisées, soit l'une ou l'autre indifféremment soit en combinaison, selon le besoin.

La chambre frigorifique 12 est munie de son côté de prises d'alimentation électrique pour pouvoir se connecter au circuit électrique du véhicule porteur 1 ou à un réseau électrique classique, cette dernière prise étant équipée des necessaires éléments redresseurs et transformateurs servant à adapter le courant aux caractéristiques de l'appareil, de sorte que cette chambre frigorifique 12 peut être maintenue en fonctionnement permanent, soit connectée au véhicule 1 lorsque ce dernier est en marche, et s'alimentant alors en 12 volts de courant continu, soit connectée à un réseau d'alimentation électrique classique de 220 volts de courant alternatif lorsqu'on est en stationnement, en supprimant ainsi le risque d'épuiser l'accumulateur du véhicule 1 .

Il est prévu que toutes les connexions de raccordement électriques et de tuyauteries entre le module 2 de l'unité en question et le véhicule porteur 1 correspondant, soient équipées de ce qu'on appelle des connecteurs ou raccords rapides, de sorte que l'installation de ladite unité sur son véhicule 1 est extrêmement facile et rapide à réaliser ; le module 2 est en outre muni, dans sa partie supérieure, d'anneaux d'accrochage 14, à raison de deux à la partie arrière et d'un, non visible, au centre de la partie avant, à l'aide desquels il est à son tour extrêmement facile de disposer ledit module 2 sur le véhicule 1 correspondant et de l'en décharger, à l'aide d'un palan ou similaire. On a prévu à cet effet que, pour faciliter l'opération de chargement et de déchargement du module, il existe un cadre en forme de harnais qui s'amarre aux anneaux d'accrochage 14, et présente un point central pour l'accrochage du palan, ce point étant calculé pour correspondre le mieux possible au centre de gravité du module.

D'un autre côté, dans le véhicule 1 qui est destiné à cette application, on prévoit au tableau de bord un panneau complémentaire à partir duquel on peut commander et manoeuvrer tout le fonctionnement de l'unité selon l'invention, de sorte que même la fermeture des portes 3 et 4 de cette unité peut être centralisée pour être commandée de l'intérieur du véhicule 1, bien que cette fermeture puisse tout aussi bien être simplement mécanique, commandée à l'aide de clés, ou qu'elle puisse être commandée par une télécommande.

Le réservoir 8 d'eau chaude peut être de son côté équipé de ses propres moyens de chauffage électrique mais, selon une autre forme de réalisation possible, on prévoit de préférence que ce réservoir comporte un échangeur de chaleur pouvant être raccordé au circuit de refroidissement du moteur du véhicule 1, et comportant un robinet de passage dans le conduit correspondant destiné à cet effet, de sorte que, outre qu'on peut ainsi profiter utilement de la chaleur produite par le moteur du véhicule 1, on obtient en même temps un moyen additionnel pour dissiper cette chaleur, en favorisant l'action du circuit de refroidissement du véhicule.

Il est évident que cette unité peut être compartimentée de différentes façons, y compris avec des portes et moyens distincts, sans que ceci n'altère en rien le principe de l'invention qui est fondé sur la réalisation d'un module rapporté, convenablement compartimenté intérieurement et muni de portes d'accès et de tous les moyens nécessaires pour que ce module puisse être installé sur la caisse d'un véhicule, en se fixant à cette caisse et qu'il puisse ainsi constituer une clinique vétérinaire mobile qui peut être amenée à n'importe quel point quelconque.

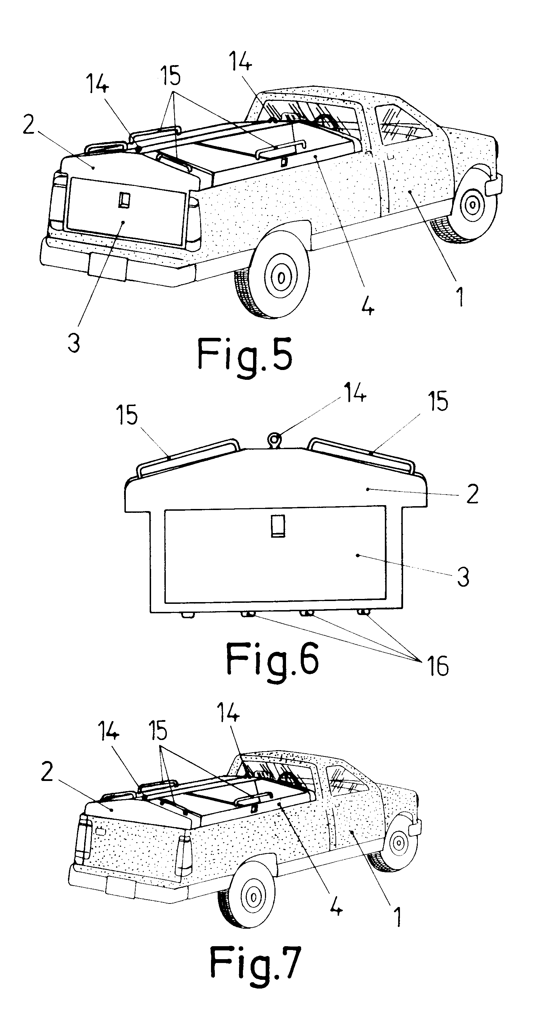

C'est ainsi qu'on a représenté sur la figure 6, comme exemple pratique possible d'une configuration différente , une unité dont le module 2 comporte dans sa partie supérieure une partie sommitale centrale plane sur laquelle sont prévus des anneaux d'accrochage 14 prévus pour le suspendre et d'où partent avec une pente sensible les portes superieures 4 qui forment ainsi des versants appropriés pour faciliter l'écoulement de l'eau de pluie vers les côtés, la surface superieure de ces portes 4 étant munie de mains-courantes périphériques 15 qui facilitent la disponibilité de ces portes, y compris la possibilité d'y charger des objets pour les transporter sur le module 2.

L'unité peut être installée sur un véhicule 1 ouvert à sa partie arrière, comme on le voit sur la figure 5, ou bien sur un véhicule 1 présentant un hayon de fermeture arrière, comme on peut le voir sur la figure 7, étant prévu que le module 2 est muni dans un cas quelconque de nervures saillantes inférieures 16 pour faciliter son appui sur la plate-forme correspondante du véhicule 1, ainsi que son coulissement pour le chargement et le déchargement ; ces nervures 16 pouvant aussi être définies en face de cannelures correspondantes de la plate-forme du véhicule 1, en permettant ainsi d'obtenir un encastrement gui facilite l'assujettissement du module 2 sur le véhicule 1 correspondant.

L'intérieur du module 2 est dans un cas quelconque convenablement isolé thermiquement, afin d'éviter les oscillations de température gui pourraient affecter les médicaments, en comportant pour cela, selon une forme de réalisation préférée, une couche de polyuréthane projetée sur le fond, tandis que, sur les portes 4 et dans le reste de l'ensemble de la carcasse, sont incorporées des plaques de polyuréthane et d'autres revêtements servant à conférer au module une caractéristique d'isothermie.

Dans l'ensemble de l'unité, on prévoit en outre l'équipement d'un convertisseur de courant capable de transformer les 12 volts de courant continu donnés par l'accumulateur du véhicule 1 en 220 volts de courant alternatif , de sorte qu'on obtient ainsi une autonomie totale, avec la possibilité d'utiliser n'importe quel appareil chirurgical électrique classique de manière que, par exemple, on puisse pratiquer des radiographies, échographies, etc..

D'un autre côté, les portes supérieures 4 sont non seulement prévues dans une configuration extérieure qui favorise l'écoulement de l'eau de pluie mais, en outre, selon une forme de réalisation préférée, ces portes 4 sont munies dans leur zone frontale d'une aile qui correspond aux côtés du véhicule de manière que, même en l'absence de rideau 13, cette aile frontale des portes 4 forme, en position d'ouverture, une couverture qui évite que des gouttes d'eau ne tombent à l'intérieur.

Bien entendu, diverses modifications et variantes pourront être apportées au dispositif qui vient d'être décrit uniquement à titre d'exemple non limitatif sans sortir du cadre de l'invention.

## Revendications

1. Unité de clinique vétérinaire mobile, caractérisée en ce qu'elle est constituée par un module (2) en forme de caisson ou d'armoire, réalisé de préférence en une matière synthétique, et qui est défini selon une configuration qui lui permet de s'adapter de façon appropriée à la zone de charge d'un véhicule porteur (1) du type camionette ou similaire, ce module présentant une portière arrière (3) rabattable à l'horizontale qui, en position ouverte, sert de table de travail, et qui ferme l'accès à une série d'objets logés à l'intérieur, qu'on peut extraire par coulissement, tandis que, dans la partie supérieure du module, sont prévues des portes latérales (4) pouvant être basculées vers le haut, lesquelles couvrent l'accès à un compartimentage intérieur dans lequel sont installés différents éléments fonctionnels, parmi lesquels se trouve une chambre frigorifique (12), cependant qu'il existe en outre dans ce volume intérieur un ample espace pour le logement d'autres éléments pouvant être extraits tels que des bacs compartimentés (5).

2. Unité de clinique vétérinaire mobile selon la revendication 1, caractérisée en ce qu'il est prévu que les portes supérieures (4) du module (2) sont munies dans leur contour et au voisinage de leur portée de fermeture, d'un type de store (13) à la façon de rideau ou similaire, qui se place automatiquement en position et peut se replier sur lui-même au cours de l'ouverture et de la fermeture des portes, pour éviter la pénétration d'eau et de salissures ambiantes lorsque lesdites portes sont ouvertes.

3. Unité de clinique vétérinaire selon la revendication 1, caractérisée en ce que, dans la structure même du module (2), sont définis des réservoirs (8) d'eau chaude et d'eau froide respectivement, auxquels un ou plusieurs tuyaux de projection d'eau (7) sont raccordés par l'intermédiaire d'une pompe électrique, cependant qu'à l'intérieur de la zone arrière accessible, sont prévus des robinets (9) pour l'ouverture et la fermeture de la sortie de l'eau provenant des réservoirs précités par le ou les tuyaux (7), ainsi qu'un interrupteur de sécurité (10) qui déconnecte l'alimentation électrique de la pompe électrique lorsqu'on ferme la portière arrière (3).

4. Unité de clinique vétérinaire mobile, selon la revendication 1, caractérisée en ce qu'à l'intérieur du module (2), est prévue une installation de chauffage capable de fonctionner sur ses moyens électriques propres ou par raccordement optionnel à l'installation de chauffage du véhicule porteur(1).

5. Unité de clinique vétérinaire mobile selon les revendications 1 et 3, caractérisée en ce que le réservoir d'eau chaude (8) est équipé d'un échangeur de chaleur pouvant se raccorder au circuit de refroidissement du moteur du véhicule, pour assurer le chauffage de l'eau de ce réservoir à l'aide de ce circuit de refroidissement.

6. Unité de clinique vétérinaire mobile selon la revendication 1, caractérisée en ce que la chambre frigorifique est équipée de prises de courant pour le raccordement au circuit électrique du véhicule ou à un réseau d'alimentation classique, la seconde prise étant équipée des éléments transformateurs et redresseurs nécessaires pour pouvoir adapter le courant à l'appareil.

7. Unité de clinique vétérinaire mobile selon la revendication 1, caractérisée en ce que le module (2) est muni, dans sa partie supérieure, d'anneaux d'accrochage (14) appropriés pour le chargement et le déchargement de ce module sur le véhicule porteur (1) au moyen d'un palan ou similaire, ou encore à l'aide d'un cadre en forme de harnais qui s'accouple aux anneaux d'accrochage (14) du module et présente un anneau d'accrochage convenablement disposé pour le palan.

8. Unité de clinique vétérinaire mobile selon la revendication 3, caractérisée en ce que, pour le tuyau d'eau (7) et pour d'autres éléments de grande longueur, tels qu'une sonde, un foetomètre, etc., il est prévu que ladite unité forme, sur toute sa longueur, un logement allongé (6), muni d'un mécanisme qui permet la rentrée automatique de ces éléments.

9. Unité de clinique vétérinaire mobile selon la revendication 1, caractérisée en ce que, dans la partie inférieure du module, sont prévues des nervures saillantes (16), de préférence disposées dans la direction longitudinale, et qui facilitent l'appui sur la plateforme correspondante du véhicule porteur et, éventuellement, le coulissement pour le chargement et le déchargement du module (2) sur ce véhicule.

10. Unité de clinique vétérinaire mobile selon la revendication 1, caractérisé en ce qu'à l'intérieur, ce module (2) est équipé d'un revêtement isolant, qui, sur le fond, peut être composé d'une couche de polyuréthane projetée et, sur le reste, de plaques incorporées de cette même matière, en donnant ainsi au module une constitution isothermique.

11. Unité de clinique vétérinaire mobile selon la revendication 1, caractérisée en ce que, dans l'ensemble de l'équipement du module (2), est prévue l'incorporation d'un convertisseur de courant capable de transformer le courant continu de 12 volts de l'accumulateur du véhicule en un courant alternatif de 220 volts, en rendant ainsi l'équipement autonome pour n'importe quel appareil électrique classique.

12. Unité de clinique vétérinaire mobile selon la revendication 1, caractérisée en ce que les portes supérieures (4) d'accès au compartimentage intérieur sont prévues selon une réalisation qui donne naissance à une aile dans la zone frontale des portes, qui correspond aux côtés du véhicule, de sorte que, dans la position d'ouverture des portes, ces ailes débordent à l'extérieur en formant ainsi une couverture qui évite la chute de gouttes d'eau de pluie à l'intérieur du module.
